# EUROPÄISCHE PATENTSCHRIFT

(11) **EP 3 150 562 B1**
(45) Veröffentlichungstag und Bekanntmachung des Hinweises auf die Patenterteilung: **16.02.2022**
(21) Anmeldenummer: 15187898.0
(22) Anmeldetag: 01.10.2015
(51) Int. Cl.: C03C 3/06, C03C 4/00

(54) **VERWENDUNG VON OPTISCHEM FILTERMATERIAL AUS DOTIERTEM QUARZGLAS SOWIE DAS OPTISCHE FILTERMATERIAL ENTHALTENDE UV-LAMPE**
USE OF OPTICAL FILTER MATERIAL MADE OF DOPED QUARTZ GLASS AND UV LAMP CONTAINING THE OPTICAL FILTER MATERIAL
UTILISATION D'UN MATÉRIAU DE FILTRE OPTIQUE EN VERRE DE QUARTZ DOPÉ ET LAMPE UV CONTENANT LE MATÉRIAU DE FILTRE OPTIQUE

(43) Veröffentlichungstag der Anmeldung: 05.04.2017
(73) Patentinhaber: Heraeus Quarzglas GmbH & Co. KG, 63450 Hanau (DE); Heraeus Noblelight GmbH, 63450 Hanau (DE)
(72) Erfinder: Stamminger, Mark, 63654 Büdingen (DE); Söller, Christoph, 63450 Hanau (DE); Schilling, Franz-Josef, 63579 Freigericht (DE); Arnold, Erich, 55127 Mainz (DE); Schötz, Gerhard, 63741 Aschaffenburg (DE); Such, Mario, 06773 Gräfenhainichen (DE); Langner, Andreas, 63579 Freigericht (DE); Roos, Björn, 63579 Freigericht (DE); Zoltner, Klaus, 63801 Kleinostheim (DE)
(74) Vertreter: Staudt, Armin Walter

(56) Entgegenhaltungen:
- EP-A2- 0 822 167
- DE-A1-102013 204 815
- DE-C- 647 537
- US-A1- 2012 148 770

## Beschreibung

### Technischer Hintergrund

Die Erfindung betrifft eine Verwendung von dotiertem Quarzglas, das zu mindestens 99 Gew.-% aus SiO₂ und Al₂O₃ besteht, wobei der Al₂O₃-Anteil im Bereich von 2 bis 4 Gew.-% liegt
Außerdem betrifft die Erfindung eine UV-Lampe mit einem Strahlaustrittsfenster aus Quarzglas für Strahlung mit Wellenlängen im ultravioletten Spektralbereich. UV-Lampen in Form von Gasentladungslampen - wie etwa Deuterium-, Halogen-, Excimer oder Quecksilberdampflampen werden beispielsweise für medizinische und therapeutische Anwendungen, in der Materialbearbeitung, zur Entkeimung und in spektroskopischen Geräten eingesetzt. Diese UV-Lampen verfügen über eine UV-Strahlenquelle zur Emission von UV-Strahlung. UV-Strahlung erfasst gemäß DIN 5031, Teil 7 den Wellenlängenbereich von 100 nm bis 380 nm. Bevor die von der UV-Strahlenquelle emittierte Strahlung auf das zu bestrahlende Gut auftrifft, passiert sie ein Strahlaustrittsfenster. Dabei handelt es sich um einen Lampenkolben, der die UV-Strahlenquelle umgibt (oder ein Teil davon) oder beispielsweise um ein Hüllrohr, das den Lampenkolben umgibt (oder ein Teil eins derartigen Hüllrohres).

### Stand der Technik

Neben der gewünschten UV-Arbeitsstrahlung enthält das Emissionsspektrum von UV-Lampen häufig auch einen Anteil an kurzwelliger ultravioletter Strahlung mit Wellenlängen unterhalb von 190 nm, die zu Ozonbildung führen und Gesundheitsschäden oder Materialalterung verursachen kann. Darüber hinaus besitzt Ozon selbst eine Absorptionsbande mit einem Maximum um 255 nm, die die Intensität der UV-Arbeitsstrahlung vermindern kann.

Quarzglas ist für UV-Strahlung über einen breiten Wellenlängenbereich transparent und daher als Material für das Strahlaustrittsfenster grundsätzlich geeignet. Die Transmission von Quarzglas ist temperaturabhängig. Im kurzwelligen Wellenlängenbereich zwischen 140 und 200 nm wird sie im Wesentlichen durch eine Absorptionskante, die sogenannte Urbachkante, bestimmt (I. T. Godmanis, A. N. Trukhin, K. Hübner "Exciton-Phonon Interaction in Crystalline and Vitreous SiO2", Phys. Stat. Sol. (b), 116 (1983), 279-287). Die Urbachkante verschiebt sich mit steigender Temperatur zu längeren Wellenlängen. Allerdings liegt sie bei den typischen Lampenbetriebstemperaturen (oberhalb von 100 °C) immer noch bei deutlich kürzeren Wellenlängen als 190 nm; sie ist also nicht geeignet, eine Ozonbildung zu verhindern.

Bei Deuteriumlampen wird deswegen häufig ein Lampenkolben aus einem speziellen Borosilikatglas eingesetzt, einem so genannten "UV-Glas", das Strahlung mit einer Wellenlänge unterhalb von 190 nm absorbiert. Borosilikatglas weist aber keinen besonders steilen Absorptionsverlauf auf, sondern dieser flacht zum langwelligeren Bereich hin ab, was die Transmission im Bereich der Arbeitswellenlänge derart vermindert, dass sich in der Regel bei einer Wellenlänge um 210 nm eine spektrale Transmission von weniger als 80% mm⁻¹ ergibt.

Deuteriumlampen emittieren UV-Strahlung im Wellenlängenbereich von etwa 180 nm bis 380 nm in Form eines kontinuierlichen, weitgehend linienfreien Spektrums und sind daher bevorzugte Strahlungsquellen für die UV-Spektroskopie. Von hochwertigen Spektralanalysegeräten wird eine hohe optische Stabilität im Sinne einer zeitlich konstanten Emission verlangt. Hier zeigen Lampenkolben aus Borosilikatglas eine weitere Schwäche. Denn ihre optische Transmission für die UV-Arbeitsstrahlung nimmt mit der Zeit merklich ab.

In dieser Hinsicht ist Quarzglas weniger anfällig; es ist außerdem einfacher zu verarbeiten als Borosilikatglas und es zeigt eine bessere Temperaturfestigkeit. Zur Verminderung der Ozonbildung ist es bei Deuteriumlampen mit einem Lampenkolben aus Quarzglas üblich, das Austrittsfenster mit einer mehrlagigen Schicht zu bedecken, die als Interferenzfilter für Wellenlängen unterhalb von 190 nm wirkt. Mittels derartiger Interferenzschichten kann eine steile Absorptionskante zwischen Sperr- und Durchlassbereich bei einer definierten Kantenwellenlänge erzeugt werden. Eine derartige UV-Lampe ist beispielsweise aus der DE 39 02 144 A1 bekannt, wobei das Strahlaustrittsfenster über einen Teil des Lampenkolben-Umfangs ausgeführt ist.

Derartige Interferenzfilter bestehen aus einer Vielzahl dünner Materialschichten. Das Aufbringen der Materialschichten auf dem Lampenkolben ist zeit- und materialaufwändig.

Es ist auch bekannt, unerwünschte Anteile der UV-Strahlung im Emissionsspektrum einer Lampe auszufiltern, indem das Glas des Strahlaustrittsfensters mit Substanzen dotiert wird, die im betreffenden Wellenlängenbereich absorbieren. So wird beispielsweise Titandioxid eingesetzt, das als Dotierstoff in Quarzglas eine Absorptionsbande mit einem Absorptionsmaximum bei etwa 200 nm erzeugt. Die WO 2010/112311 A1 schlägt stattdessen oder ergänzend dazu ein Filtermaterial aus einem mit Galliumoxid dotierten Quarzglas vor.

Bei den mit Titanoxid oder Galliumoxid dotierten Quarzgläsern liegt die Kantenwellenlänge λ_{c} im Wellenlängenbereich von 230 bis 250 nm. Sie sind als Filtermaterial für eine Arbeitsstrahlung mit einer Wellenlänge unterhalb von 230 nm nicht geeignet.

Die DE 10 2013 204 815 A1 offenbart Seltenerd-dotierte Faserkerne in Faserlasern. Die Brechzahlanhebung infolge der Seltenerd-Dotierung soll durch Co-Dotierung mit Fluor kompensiert werden. Die Verwendung des dotierten Quarzglases für optische Anwendungen auch als Filterglas wird erwähnt. Bei allen Ausführungsbeispielen enthält das dotierte Quarzglas Seltenerdmetalloxid und Al₂O₃ in unterschiedlichen Mol-Anteilen, die (umgerechnet in Gew.-% und ohne Berücksichtigung der Seltenerddotierung und des Fluorgehalts) unter 2 und über 5 Gew.-% liegen.

Die EP 0 822 167 A2 befasst sich mit einer Vorform für optische Quarzglas-Fasern mit einem Al₂O₃-dotierten Faserkern. Der homogen dotierte Faserkern kann bis zu 1,3 Gew.-% Al₂O₃ enthalten; darüber hinausgehende Mengen führen zur Kristallisation beim Faserziehen. Faserkerne mit einem zentralen Dip in der Al₂O₃-Konzentration können außerhalb ihrer "centerline" auch höhere Al₂O₃-Gehalte bis 2,35 Gew.-% aufweisen.

In der DE 647 537 C werden Übergangsgläser für die Herstellung einer Einschmelzung von Wolfram- oder Molybdän-Stromzuführungen in Lampenkolben beschrieben. Die Gläser enthalten 65-96 % SiO₂ und zwischen 4-20 % Al₂O₃ und optional weitere Oxide. Es wird ein Satz von zehn Übergangsgläsern angegeben, deren Ausdehnungskoeffizient sich stufenweise erhöht.

Die US 2012/0148770 A1 beschreibt Verpackungsglas für pharmazeutische Produkte. Das Glas enthält zwischen 82 bis 99,9999 Gew.-% SiO₂; der Rest sind Dotierstoffe, darunter Al₂O₃. Das Al₂O₃-dotierte Quarzglas zeichnet sich durch hohe chemische Beständigkeit und eine moderate Viskosität aus.

### Technische Aufgabenstellung

In Anbetracht der oben erläuterten Schwächen bisheriger Filtermaterialien für Strahlaustrittsfenster von UV-Lampen wäre der Einsatz von dotiertem Quarzglas an und für sich eine geeignete Maßnahme, um kostengünstig und reproduzierbar eine Filterwirkung zu erreichen.

Allerdings stehen geeignete Dotierstoffe nicht ohne weiteres zur Verfügung, die die gewünschte Absorption erzeugen, ohne dass sie sich gleichzeitig auf die Transparenz für die Arbeitsstrahlung auswirken. Außerdem kann die Dotierung ungewollte Veränderungen der Quarzglaseigenschaften hervorrufen, insbesondere kann sie die Strahlungsresistenz gegenüber UV-Strahlung verringern. Letzteres ist gerade bei Quarzglas für UV-Lampen besonders nachteilig, denn UV-Strahlungsschäden bewirken eine allmähliche Abnahme der UV-Transmission (Alterung) und damit eine nachlassende UV-Emission.

Der Erfindung liegt daher die Aufgabe zugrunde, eine Verwendung von dotiertem Quarzglas anzugeben, bei dem die Dotierung eine technische Wirkung als optisches Filtermaterial hat, mit einer möglichst hohen Transparenz für Arbeitsstrahlung im ultravioletten Spektralbereich oberhalb von 210 nm bei gleichzeitig geringer Transparenz im Wellenlängenbereich unterhalb von etwa 190 nm.

Weiterhin liegt der Erfindung die Aufgabe zugrunde, eine UV-Lampe mit einem Strahlaustrittsfenster anzugeben, das derartiges Filtermaterial enthält, und die ein effektives Bestrahlen einer Oberfläche, einer Flüssigkeit oder eines Gases mittels UV-Strahlung gewährleistet, wobei gleichzeitig eine möglichst geringe Ozonbildung auftritt.

### Zusammenfassung der Erfindung

Hinsichtlich der Verwendung wird diese Aufgabe durch die in Anspruch 1 definierte Verwendung gelöst.

Unter Quarzglas wird hier hochkieselsäurehaltiges Glas mit einem SiO₂-Anteil von mindestens 93 Gew.-% verstanden. Es hat sich überraschend gezeigt, dass die Dotierung des Quarzglases mit Al₂O₃ (Aluminiumoxid) eine Verschiebung der Urbachkante zu längeren Wellenlängen hin bewirkt, ohne dass sich dies auf die Kantensteilheit und die Transmission des dotierten Quarzglases im UV-Wellenlängenbereich von etwa 200 nm bis 250 nm nennenswert auswirkt. Diese bachkante zu längeren Wellenlängen hin bewirkt, ohne dass sich dies auf die Kantensteilheit und die Transmission des dotierten Quarzglases im UV-Wellenlängenbereich von etwa 200 nm bis 250 nm nennenswert auswirkt. Diese Wirkung macht sich jedoch erst bei relativ hohen Al₂O₃-Konzentrationen von etwa 1 Gew.-% und mehr bemerkbar. Dies erklärt, dass der Effekt in der Literatur bisher nicht beschrieben ist, obgleich Aluminiumoxid ein gängiger Dotierstoff von Quarzglas ist, da es sich in niedriger Konzentration im ppm-Bereich versteifend auf die Netzwerkstruktur von Quarzglas auswirkt und dadurch die Viskosität erhöht und die thermische Beständigkeit verbessert.

Bei hohen Konzentrationen von 1,5 Gew.-% und mehr zeigt Aluminiumoxid in Quarzglas überraschenderweise eine Absorption im UV-Wellenlängenbereich, die sich als steile Absorptionskante bemerkbar macht. Die sogenannte Kantenwellenlänge λc liegt je nach Aluminiumoxid-Konzentration des Quarzglases im Wellenlängenbereich von etwa 170 bis 200 nm, wobei die Absorptionskante mit zunehmender Aluminiumoxid-Konzentration längerwelliger wird.

Dabei bleibt die Absorptionszunahme für Wellenlängen um 200 nm und oberhalb davon gering, so dass für die übliche UV-Arbeitsstrahlung bis etwa 380 nm eine hohe Lichtdurchlässigkeit erhalten bleibt. Als Maß dafür kann die spektrale Transmission bei der Wellenlänge von 210 nm dienen, die für das erfindungsgemäße Filtermaterial vorzugsweise 80% mm⁻¹ oder höher ist. Dieser Mindestwert für die Transmission dient lediglich als Maß für hohe Transparenz im UV-Wellenlängenbereich von 200 bis 380 nm.

Das Filtermaterial aus Aluminiumoxid-dotiertem Quarzglas zeichnet sich somit durch ein spektrales Transmissionsprofil aus, das einerseits eine effektive Bestrahlung im UV-Wellenlängenbereich ermöglicht und andererseits UV-Strahlung mit Wellenlängen <190 nm weitgehend absorbiert. Dies wird durch die Lage der Kantenwellenlänge λ_{c} und die Steilheit der Absorptionsflanke erreicht, die sich infolge der Dotierung des Quarzglases mit Aluminiumoxid ergibt.

Das Filtermaterial dient zur Herstellung eines Filterelements in Form eines Bauteils oder in Form einer Beschichtung auf einem UV-durchlässigen Substrat. Die Wirkung der Aluminiumoxid-Dotierung hängt von der Konzentration des Aluminiumoxids und der durchstrahlten Schichtdicke ab. Bei geringer Schichtdicke, wie etwa bei einer aufgedampften oder aufgesputterten Schicht mit Dicken im µm-Bereich, sind vergleichsweise hohe Konzentrationen an Aluminiumoxid erforderlich, um eine nennenswerte Absorption zu bewirken; und geringe Konzentrationen bei einer großen Schichtdicke.

Für übliche Filterschichtstärken im Dickenbereich von etwa 0,5 bis zu wenigen Millimetern liegt erfindungsgemäß der Al₂O₃-Anteil im Bereich von 2 bis 4 Gew.%.

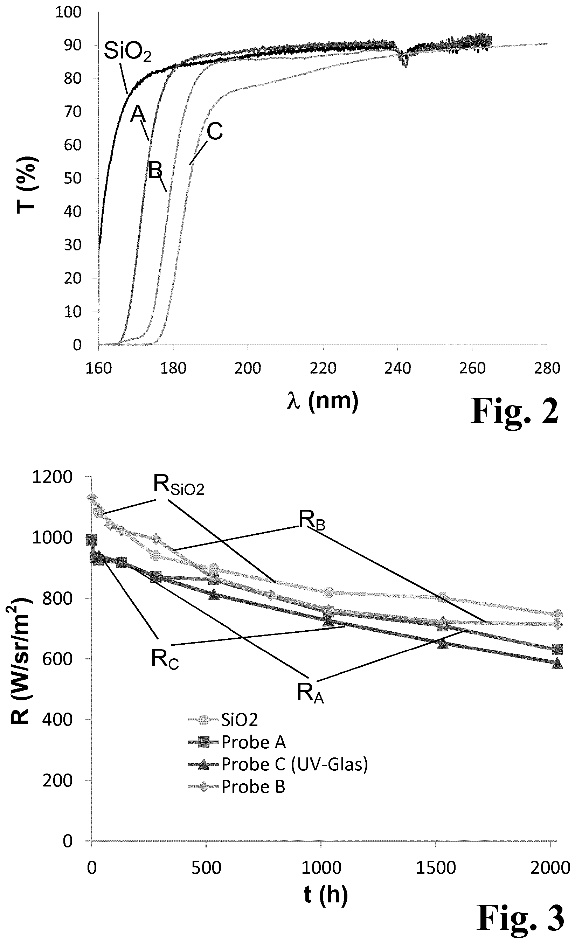

So ergeben beispielsweise eine Aluminiumoxid-Konzentration von etwa 2,7 Gew.% und eine durchstrahlte Materialschichtdicke von 1 mm eine spektrale Lage der Absorptionskante bei einer Wellenlänge um 180 nm (siehe Figur 2), aber mit dem Vorteil einer im Vergleich zu Borosilikatglas steileren Flanke der Absorptionskante.

Mit zunehmender Aluminiumoxid-Konzentration verschiebt sich die Absorptionskante weiter in Richtung 200 nm. Soll eine nahezu vollständige Absorption kurzwelliger UV-Strahlung im Wellenlängenbereich unterhalb von 190 nm erreicht werden, sind Konzentrationen an der oberen Bereichsgrenze bevorzugt. Bei sehr hohen Aluminiumoxid-Konzentrationen (mehr als etwa 6 Gew.-%) ergibt sich eine zunehmende Absorption auch im längerwelligen Bereich.

Mindestens der SiO₂-Anteil des dotierten Quarzglases ist synthetisch erzeugt.

Die oxidative oder hydrolytische Synthese von SiO₂ aus dampfförmigen oder flüssigen Ausgangssubstanzen mittels Gasphasenabscheidung oder Sol-Gel-Technik ist allgemein bekannt. Synthetisch erzeugtes Quarzglas zeichnet sich durch eine hohe Transmission im Bereich der typischen UV-Arbeitswellenlängen sowie durch eine hohe UV-Strahlenbeständigkeit aus. Allerdings hat synthetisch erzeugtes Quarzglas häufig einen hohen Hydroxylgruppengehalt, der zu einer Verringerung der thermischen Beständigkeit des Quarzglases führen kann. Um diesen Nachteil zu vermindern, zeichnet sich das dotierte Quarzglas bevorzugt durch einen Hydroxylgruppengehalt von weniger als 200 Gew.-ppm, vorzugsweise weniger als 10 Gew.-ppm, aus.

Das optische Filtermaterial ist geeignet, den kurzwelligen Anteil der UV-Emission einer UV-Strahlenquelle zu vermindern und so die Bildung von Ozon zu vermeiden. Es eignet sich insbesondere für die Herstellung von Lampenmaterial für UV-Lampen, wie etwa als Lampenrohr oder als Hüllrohr. Eine hohe Emission und Transparenz im Wellenlängenbereich von etwa 210 bis 380 nm ist insbesondere für Deuteriumlampen relevant, die eine besonders bevorzugte Anwendung für das Filtermaterial darstellen.

Hinsichtlich der UV-Lampe wird die angegebene Aufgabe ausgehend von einer UV-Lampe der eingangs genannten Gattung erfindungsgemäß dadurch gelöst, dass im Strahlengang ein Filtermaterial aus dotiertem Quarzglas vorgesehen ist, das zu mindestens 99 Gew.-% aus SiO₂ und Al₂O₃ besteht, wobei der Al₂O₃-Anteil im Bereich von 2 bis 4 Gew.-% liegt, dessen Kantenwellenlänge bei einer Wellenlänge unterhalb von 190 nm liegt, und dessen spektrale Transmission bei der Wellenlänge von 210 nm 80% mm-1 oder höher ist.

Der Strahlengang der UV-Lampe wird durch den geometrischen Verlauf der Arbeitsstrahlung ausgehend von der eigentlichen Lichtquelle bis zum Strahlaustritt aus der Lampe definiert. In diesem Strahlengang ist Filtermaterial gemäß der Erfindung enthalten. Die obigen Erläuterungen zum Filtermaterial gelten somit auch für das Filtermaterial im Strahlengang der UV-Lampe.

Insbesondere hat es sich überraschend gezeigt, dass eine hohe Dotierung mit Aluminiumoxid im Bereich von 2 bis 4 Gew.-% eine Verschiebung der Urbachkante von reinem Quarzglas zu längeren Wellenlängen bewirkt. Dabei bleibt für die üblichen UV-Arbeitswellenlängen bis etwa 380 nm eine hohe Lichtdurchlässigkeit des dotierten Quarzglases erhalten. Als Maß dafür kann die spektrale Transmission des Filtermaterials bei der Wellenlänge von 210 nm dienen, die vorzugsweise 80% mm⁻¹ oder höher ist.

Das Filtermaterial liegt im Strahlengang als Schicht oder separates Bauteil vor, wie etwa als Filter, oder es bildet einen Lampenkolben oder ein Hüllrohr der UV-Lampe oder einen Teil davon. Die Deuteriumlampe ist eine bevorzugte Ausführungsform der UV-Lampe.

Die Wirkung der Aluminiumoxid-Dotierung im Hinblick auf die Verminderung der Emission des kurzwelligen UV-Anteils der UV-Strahlenquelle und die Verminderung von Ozon- Bildung hängt von der Konzentration des Aluminiumoxids und der durchstrahlten Schichtdicke ab. Bei geringer Schichtdicke, wie etwa bei einer aufgedampften oder aufgesputterten Schicht mit Dicken im µm-Bereich, sind vergleichsweise hohe Konzentrationen an Aluminiumoxid erforderlich, um eine nennenswerte Absorption zu bewirken; und geringe Konzentrationen bei einer großen Schichtdicke.

Mindestens der SiO₂₋Anteil des Aluminiumoxid-dotierten Quarzglases ist bevorzugt synthetisch erzeugt. Synthetisch erzeugtes Quarzglas zeichnet sich durch eine hohe Transmission im Bereich der typischen UV-Arbeitswellenlängen sowie durch eine hohe UV-Strahlenbeständigkeit aus. Unter Umständen kann der Aluminiumoxid-Anteil des Quarzglases jedoch zur Bildung von Sauerstoffdefizitdefekten führen. Einige dieser Sauerstofffehlstellen, die so genannten "ODC I-Defekte" ("oxygen deficiency defects") zeigen bei einer Wellenlänge von 164 nm eine Absorptionsbande. Diese Absorption kann in Kombination mit der Aluminium-oxid-Dotierung des Quarzglases die spektrale Lage der Absorptionskante beeinflussen. Es ist auch bekannt, dass sich ODC I-Defekte unter UV-Bestrahlung in andere strukturelle Defekte umwandeln können, insbesondere in sogenannte"E'-Zentren", einem Siliciumatom mit einem ungepaarten Elektron. "E'-Zentren" zeigen jedoch eine starke Absorption bei einer Wellenlänge um 215 nm, welche die Transparenz des Aluminiumoxid-dotierten Filtermaterials für die UV-Arbeitsstrahlung verringert. Überraschenderweise tritt diese unerwünschte Umwandlung beim Aluminiumoxid-dotierten Filtermaterial jedoch nicht oder in einem vergleichsweise geringeren Ausmaß ein, insbesondere wenn es in einer Deuterium lampe eingesetzt wird. Eine mögliche Erklärung dafür wird weiter unten noch näher erläutert.

Synthetisch erzeugtes Quarzglas weist häufig einen hohen Hydroxylgruppengehalt auf, der zu einer Verringerung der thermischen Beständigkeit des Aluminiumoxid-dotierten Quarzglases führen kann. Um diesen Nachteil zu vermindern, weist das Quarzglas für das Filtermaterial vorteilhafterweise einen Hydroxylgruppengehalt von weniger als 200 Gew.-ppm, vorzugsweise weniger als 10 Gew.ppm, auf.

Bei einer besonders bevorzugten Ausführungsform weist die UV-Lampe ein Strahlaustrittsfenster auf, das aus dem Aluminiumoxid-dotierten Quarzglas besteht oder das mit dem Aluminiumoxid-dotierten Quarzglas beschichtet ist.

Die Lebensdauer der UV-Lampe wird dabei vom Material des Strahlaustrittsfensters beeinflusst, aber auch von einem Effekt, der als "Gasaufzehrung" bekannt ist. Dieser Effekt tritt insbesondere bei Deuteriumlampen auf und beruht darauf, dass Komponenten aus dem Lampenfüllgas oder den Elektroden in den Lampenkolben eindiffundieren - und zwar bevorzugt in das besonders heiße Strahlaustrittsfenster - so dass sich die chemische Zusammensetzung des Strahlaustrittsfensters allmählich verändert.

Um diesem Effekt entgegenzuwirken, hat es sich als vorteilhaft erwiesen, wenn das Strahlaustrittsfenster auf seiner einem Lampen-Innenraum zugewandten Seite mit einer Schicht aus Aluminiumoxid belegt ist.

Die Schicht aus Aluminiumoxid wirkt als Barriere gegen die Eindiffusion von Komponenten aus dem Füllgas oder den Elektroden in das Material des Strahlaustrittsfensters; sie vermindert beispielsweise die Reaktion von etwaiger Beschichtungsmasse auf der Kathode mit dem Quarzglas des Strahlaustrittsfensters. Vorzugsweise enthält die Diffusionsbarriereschicht amorphes Aluminiumoxid, hat eine Dicke im Bereich von 20 bis 200 nm und ist für UV-Strahlung optisch transparent.

Die erfindungsgemäße UV-Lampe emittiert Arbeitsstrahlung, die die Wellenlänge von 210 nm umfasst. Sie wird beispielsweise zum Bestrahlen einer Oberfläche, einer Flüssigkeit oder eines Gases eingesetzt, wobei Lebensmittel, Luft, Flüssigkeiten oder die Oberflächen technischer Güter effektiv entkeimt, Lack- oder Kunststoffschichten gehärtet, und medizinische, zahnmedizinische oder therapeutische Bestrahlungen oder spektroskopische Messungen an Gasen oder Flüssigkeiten vorgenommen werden. Die Arbeitsstrahlung wird entlang eines Strahlengangs auf das zu behandelnde, zu bearbeitende oder zu messende Objekt geleitet und durchläuft dabei das Filtermaterial aus dotiertem Quarzglas, das sich im Strahlengang befindet.

Der spektrale Transmissionsverlauf des mit Aluminiumoxid dotierten Quarzglases zeigt je nach Konzentration des Dotierstoffs eine S-förmige Absorptionskante mit einem 50%-Wert (bezogen auf die maximale Transmission) im Bereich von 170 nm bis etwa 200 nm. Diese Absorptionskante wird auch als "Urbachkante" bezeichnet. Sie kann mit steigender Temperatur des Strahlaustrittsfensters zu längeren Wellenlängen verlagert werden. Dabei bewirkt eine Temperaturerhöhung von 100 °C in etwa eine Verlagerung um 2 bis 3 nm. Dadurch, dass das Strahlaustrittsfenster auf einer Betriebstemperatur im Bereich von 250 bis 350 °C gehalten wird, kann die Urbachkante unabhängig vom Verlagerungseffekt durch den Aluminiumoxid-Dotierung um weitere etwa 6 bis 9 nm in den langwelligen Bereich verschoben und die Ozonbildung weiter verringert werden. Bei Kenntnis der nominalen Betriebstemperatur während des Verfahrens kann dieser Effekt bei der Bemessung der Dotierstoffkonzentration berücksichtigt werden, wenn es auf eine exakte Position der Urbachkante ankommt.

### Ausführungsbeispiel

Nachfolgend wird die Erfindung anhand von Ausführungsbeispielen und einer Zeichnung näher erläutert. Unter diesen Beispielen sind: Probe A ein Referenzbeispiel, Probe C ein Vergleichsbeispiel und Probe B erfindungsgemäß.

Dabei zeigt
- **Figur 1**: eine schematische Darstellung einer erfindungsgemäßen UV-Lampe in der Ausführungsform einer Deuteriumlampe anhand eines Querschnitts,
- **Figur 2**: ein Diagramm mit Transmissionskurven verschiedener Aluminiumoxiddotierter Quarzgläser im Vergleich zu undotiertem Quarzglas und einem handelsüblichen UV-Glas (Borosilikatglas), und
- **Figur 3**: ein Diagramm zum zeitlichen Verlauf der gemessenen Strahldichte im Spektralbereich von 205 bis 215 nm für ein Aluminiumoxid-dotiertes Quarzglas im Vergleich zu einem handelsüblichen UV-Glas (Borosilikatglas).

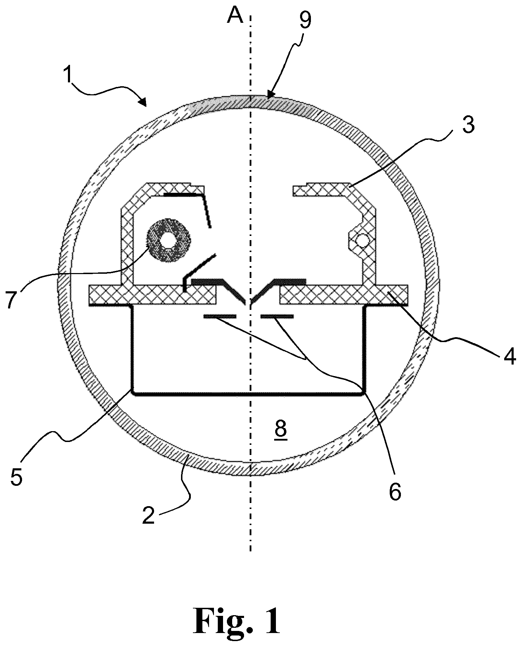

In **Figur 1** ist eine Deuteriumlampe 1 im Querschnitt schematisch entlang der optischen Achse A dargestellt. Die Lampe 1 weist einen Lampenkolben 2 auf, der einen mit Deuterium gefüllten Innenraum 8 umschließt. In dem Innenraum 8 findet sich ein zweiteiliges Gehäuse, das aus einem vorderen Gehäuseteil 3 mit darin angeordneter Kathode 7, einer Gehäusezwischenwand 4 aus Aluminiumoxid sowie aus einem hinteren Gehäuseteil 5 aus Metall besteht, in dem sich eine Anode 6 befindet. Am vorderen Gehäuseteil 3 ist eine Lichtaustrittsöffnung 9 vorgesehen, die in Figur 1 durch Grauschattierung hervorgehoben ist. Dieser Bereich des Lampenkolbens 2 repräsentiert ein "Strahlaustrittsfenster" im Sinne der Erfindung. Wird zwischen Kathode 7 und Anode 6 eine Entladung gezündet, so gelangt ein Lichtkegel entlang der optischen Achse A auf die Lichtaustrittsöffnung 9.

Der gesamte Lampenkolben 2 einschließlich seines als Strahlaustrittsfenster 9 dienenden Umfangsabschnitts besteht aus synthetisch erzeugtem Quarzglas, das homogen mit Aluminiumoxid in einer Konzentration von 2,7 Gew.-% dotiert ist.

Die dem Innenraum 8 zugewandte Innenseite des Lampenkolbens 2 ist vollständig mit einer (nicht eingezeichneten) Diffusionssperrschicht aus amorphem Al₂O₃ beschichtet. Die Diffusionssperrschicht wird durch Tauchbeschichtung im Sol-Gel-Tauchverfahren aufgebracht, getrocknet und 12 h bei 900 °C eingebrannt. Danach ist sie im Wellenlängenbereich zwischen 100 und 1100 nm transparent und hat eine Dicke von 100 nm. Die Diffusionssperrschicht vermindert die Ausdiffusion von Komponenten des Füllgases und der Elektrodenbeschichtung und deren Reaktion mit dem Material des Lampenkolbens 2 und zwar insbesondere im Bereich der beim Lampenbetrieb besonders heißen Lichtaustrittsöffnung 9.

Die Aluminiumoxid-Dotierung des Lampenkolbens 2 bewirkt eine Verschiebung der Urbachkante des Quarzglases, wie die Transmissionsspektren von **Figur 2** eindrucksvoll zeigen. Auf der y-Achse ist die gemessene Transmission T (in %) über die Wellenlänge λ (in nm) über den Wellenlängenbereich von 160 bis etwa 280 nm aufgetragen. Die "gemessene Transmission" T beinhaltet Reflexionsverluste an Oberflächen und unterscheidet sich insoweit von der sogenannten "inneren Transmission" (oder "Reintransmission").

Das Diagramm enthält Transmissionsprofile von zwei Filtermaterialproben aus SiO₂-Al₂O₃-Glas, die sich in ihrem Aluminiumoxid-Gehalt voneinander unterscheiden (Probe A: 98,9 SiO₂ /1,1 Al₂O₃; Probe B: 97,3 SiO₂ /2,7 Al₂O₃). Zum Vergleich sind außerdem der Transmissionsverlauf eines handelsüblichen synthetisch erzeugten und undotierten Quarzglases (Kurve SiO₂: Handelsname "Suprasil 1") sowie der Transmissionsverlauf eines handelsüblichen Borosilikatglases (Kurve C; Handelsname: Schott 8337B) eingetragen.

In der folgenden Tabelle 1 sind für die Messproben die Kantenwellenlänge λ_{c} bei Raumtemperatur und der bei der Wellenlänge 210 nm ermittelte Transmissionswert T(210nm) sowie die jeweilige Probendicke zusammengefasst.

**Tabelle 1**

| Probe / Kurve | λc [nm] | T(210nm) [%] | Probendicke [mm] |
|---|---|---|---|
| SiO₂ | 163 | 88 | 1 |
| A | 172 | 89 | 1 |
| B | 179 | 86 | 1 |
| C | 183 | 79 | 0,3 |

Aus den Transmissionskurven A und B ist ersichtlich, dass die Dotierung mit Aluminiumoxid eine Verschiebung der Kantenwellenlänge λ_{c} gegenüber reinem SiO₂ bewirkt. Eine Dotierstoffkonzentration von 1,1 Gew.-% (Probe A) ergibt eine Verschiebung der Absorptionskante um etwa 9 nm. Mit zunehmender Dotierstoffkonzentration verschiebt sich die Absorptionskante weiter zu längeren Wellenlängen, ohne dass sich die Steilheit der Absorptionskante und der hier als repräsentativ ausgewählte Transmissionswert T(210nm) wesentlich verändern.

Bei einer Al₂O₃-Konzentration von 2,7 Gew.-% (Kurve B) zeigt die Absorptionskante des dotierten Quarzglases bei einer durchstrahlten Materialschichtdicke von 1 mm eine spektrale Lage bei einer Wellenlänge um 179 nm, also etwas kurzwelliger als die des Borosilikat-UV-Glases (Kurve C) bei einer durchstrahlten Materialschichtdicke von 0,3 mm. Die Absorptionskante ist bei Kurve B jedoch etwas steiler als bei Kurve C und insbesondere liegt der repräsentative Transmissionswert T(210nm) mit 86% höher als bei dem UV-Glas (etwa 79%). In dem Zusammenhang ist zu beachten, dass die Messdicke der Probe C nur 0.3 mm beträgt. Die geringe Messdicke ist erforderlich, um einen mit den anderen Proben vergleichbaren Transmissionswert im repräsentativen Wellenlängenbereich (beispielsweise T(210nm)) zu erreichen, der die Ozonbildung vermindert.

Bei einer Aluminiumoxid-Konzentration von 4 Gew.-% (im Diagramm nicht enthalten) zeigt sich eine weitere Verschiebung der Urbachkante, wobei die Transmission für den Wellenlängenbereich < 180 nm auf Werte unterhalb von 5 % reduziert wird. Infolge der vergleichsweise hohen Dotierstoffkonzentration verläuft die Absorptionskante zwar ein wenig flacher als beim UV-Glas von Probe C, erreicht aber dennoch eine höhere maximale Transmission mit einem T(210nm)-Wert um 80 %.

Um die Langzeitstabilität des aluminiumoxid-dotierten Quarzglases unter typischen Betriebsbedingungen einer Deuterium-Entladungslampe zu testen, wurden aus den Werkstoffen der Proben A und B von Tabelle 1 jeweils Fenster in der Größe des Strahlaustrittsfensters 9 gefertigt und im Bereich des Strahlaustrittsfensters 9 in den Lampenkolben 2 aus undotiertem Quarzglas dicht eingeschmolzen. Die Innenseite des Lampenkolbens 2 wurde daraufhin mit einer Schicht aus amorphem Al₂O₃ versehen, wie dies oben erläutert ist, und der Lampenkolben wurde wieder mit dem nominalen Füllgas befüllt. Die so modifizierte Deuteriumlampe wurde 2000 Stunden lang unter in einem für die Flüssigkeitschromatographie gebräuchlichen Temperierkörper betrieben und dabei von Zeit zu Zeit an einem kalibrierten Messstand spektral aufgelöst die Strahldichte gemessen.

Das Diagramm von **Figur 3** zeigt das Ergebnis dieser Messung im Vergleich zu einer Deuteriumlampe mit einem Lampenkolben aus dem handelsüblichen Borosilikatglas (Schott 8337B; Kurve R_{c}) und einem Lampenkolben aus dem undotiertem, synthetisch erzeugten SiO₂ Glas (Suprasil 1; Kurve: R_{SiO2}) mit einer Beschichtung aus amorphem Aluminiumoxid. Auf der y-Achse ist die spezifische Strahldichte R (als Absolutwert) im Spektralbereich zwischen 205 und 215 nm (in W/sr/m²) (Watt pro m² und pro Steradiant) als Funktion der Lampenbetriebsdauer t (in h) aufgetragen.

Daraus ist erkennbar, dass die gemessene Strahldichte R bei allen Lampen zunächst etwa gleichlaufend abnimmt. Den geringsten Verlust an Strahldichte zeigt die Lampe mit Lampenkolben aus reinem SiO₂ (jedoch verbunden mit Ozonbildung). Bei der handelsüblichen Deuteriumlampe aus UV-Glas (Kurve R_{C}) und bei der UV-Lampe mit Lampenkolben aus 98,9 SiO₂ /1,1 Al₂O₃ (Kurve R_{A}) sinkt die gemessene Strahldichte kontinuierlich, insbesondere auch während der Zeitspanne zwischen 1000 und 1500 Betriebsstunden, wohingegen sich bei der mit aluminiumoxiddotiertem Quarzglasfenster 97,3 SiO₂/2,7 Al₂O₃ (Kurve R_{B}) ausgestatteten Deuteriumlampe die gemessene Strahldichte zwischen 1000 und 1500 Betriebsstunden auf etwa konstantem Niveau einzupendeln scheint.

Insbesondere das Quarzglas von Probe B ist besonders geeignet als Filtermaterial oder Lampenkolben für eine Lichtquelle in einem Spektralphotometer eingesetzt zu werden. Ihre (gemessene) spektrale Transmission bei der Wellenlänge von 210 nm liegt bei etwa 86 % und bei 190 nm bei etwa 84%. Die Kantenwellenlänge λ_{c} liegt bei Raumtemperatur bei etwa 179 nm und wird durch Temperierung des Lampenkolbens auf eine Temperatur von 300 °C auf etwa 185 bis 188 nm verschoben, so dass sich eine geringe Ozonbildung ergibt.

Nachfolgend wird die Herstellung des Filtermaterials aus aluminiumoxiddotiertem Quarzglas anhand eines Beispiels erläutert:
Es wird ein Schlicker aus diskreten SiO₂-Partikeln mit einer mittleren Teilchengröße um 10 µm in Reinstwasser hergestellt. Eine Menge von 285,7g des Schlickers mit einer Restfeuchte von 37,4% wird mit 1000ml Reinstwasser verdünnt. Durch Zugabe einer konzentrierten Ammoniaklösung in einer Menge von 75 ml wird ein pH-Wert von 14 eingestellt. Die alkalische Suspension wird homogenisiert und durch einen 25 µm-Membranfilter abfiltriert.

Es wird eine wässrige Lösung aus 62,71g AlCl₃ in 400 ml Reinstwasser erzeugt und ebenfalls durch einen 15 µm-Membranfilter abfiltriert. Anstelle von AlCl₃ können auch andere aluminiumhaltige Ausgangssubstanzen eingesetzt werden, wie beispielsweise organische Al-Verbindungen, Nitride oder Fluoride.

Der durch Rühren bewegten Suspension wird die Dotierstofflösung in Form eines Sprühnebels während einer Dauer von 65 Minuten zugeführt. Zur Erzeugung des Sprühnebels wird die Dotierstofflösung mittels einer Sprühdüse zerstäubt, wobei ein Arbeitsdruck von 2 bar und eine Durchflussrate von 0,8 l/h eingestellt wird. Der so erzeugte Sprühnebel enthält Tropfen mit einem mittleren Durchmesser zwischen 10 µm und 40 µm. Anschließend wird der dotierte Schlicker weitere 2 Stunden lang durch Rühren homogenisiert. Mit dieser Vorgehensweise ist gewährleistet, dass ein optimal homogen dotierter SiO₂-Schlicker erhalten wird.

Der dotierte SiO₂-Schlicker wird eingefroren und durch sogenannte Frostgranulation zu einem Granulat weiterverarbeitet. Dabei wird der nach dem Auftauen erhaltene Granulatschlamm mehrmals mit Reinstwasser gewaschen und das überschüssige Wasser jeweils abdekantiert.

Anschließend wird der von Ammoniak befreite und gereinigte Granulatschlamm bei einer Temperatur um 400 °C 6 Stunden lang getrocknet. Das getrocknete Granulat wird in eine Kunststoffform eingeschweißt und bei 400bar isostatisch gepresst.

Der so erhaltene Granulat-Pressling wird unter Heliumspülung erhitzt und danach etwa 8 Stunden lang in chlorhaltiger Atmosphäre bei etwa 900 °C behandelt. Dadurch werden Verunreinigungen aus dem Pressling entfernt und der Hydroxylgruppengehalt wird auf weniger als 200 Gew.-ppm, bevorzugt weniger als 10 Gew.-ppm reduziert. Der gereinigte Granulatpressling wird abschließend vorgesintert, indem er in einem Vakuumofen (< 1 mbar) bis auf eine Temperatur von 1550 °C erhitzt wird. Das Sintern zu einem Körper aus transparentem Glas erfolgt unter Argon-Atmosphäre durch Gasdrucksintern. Während einer ersten Phase von neun Stunden, die das Aufheizen und die ersten drei Stunden der Haltezeit bei dieser Temperatur umfasst, wird im Sinterofen ein Vakuum (< 5 mbar) aufrechterhalten, unterbrochen von Inertgas-Spülvorgängen. Während einer zweiten Phase wird ein Argonüberdruck von 15 bar erzeugt und die Ofentemperatur auf 1680 °C erhöht. Bei dieser Temperatur wird der vorgesinterte Körper während einer Dauer von 1 h zu einem Block aus transparentem Quarzglas verglast und anschließend abgekühlt.

### Definitionen

### Strahlengang

Der Strahlengang der UV-Lampe wird durch den geometrischen Verlauf der Arbeitsstrahlung ausgehend von der eigentlichen Lichtquelle bis zum Strahlaustritt aus der Lampe definiert. Der Strahlengang umfasst ein Strahlaustrittsfenster, das die von der UV-Lampe emittierte Strahlung passiert, ehe sie auf das zu bestrahlende Gut auftrifft.

### Quarzglas

Unter Quarzglas wird hier hochkieselsäurehaltiges Glas mit einem SiO₂-Anteil von mindestens 93 Gew.-% verstanden.

### Kantenwellenlänge

Die Kantenwellenlänge λ_{c} - hier auch als Absorptionskante bezeichnet - entspricht derjenigen Wellenlänge, bei der der spektrale Reintransmissionsgrad die

Hälfte der Maximaldifferenz der Reintransmissionsgrade von Sperr- und Durchlassbereich beträgt.

### Transmission

Der spektrale Reintransmissionsgrad (auch als "innere Transmission bezeichnet) ist abstrahiert von Reflexionsverlusten und versteht sich per Definition als das Verhältnis des ausdringenden spektralen Strahlungsflusses zu dem eingedrungenen Strahlungsfluss.

Im Unterschied dazu beinhaltet die "gemessene Transmission" Reflexionsverluste an Oberflächen.

### Spektrale Strahldichte

Die spektrale Strahldichte eines Körpers ist in DIN EN ISO 9288 definiert. Sie gibt an, welche Strahlungsleistung der Körper bei einer Frequenz in die durch einen Polarwinkel und den Azimutwinkel gegebene Richtung pro projizierter Fläche, pro Raumwinkel und pro Frequenzbreite aussendet.

## Patentansprüche

1. Verwendung von dotiertem Quarzglas, das zu mindestens 99 Gew.-% aus SiO₂ und Al₂O₃ besteht, wobei der Al₂O₃-Anteil im Bereich von 2 bis 4 Gew.-% liegt, als optisches Filtermaterial, das eine spektrale Transmission bei der Wellenlänge von 210 nm von 80% mm⁻¹ oder höher und eine Kantenwellenlänge aufweist, zum Zweck der Verschiebung der Kantenwellenlänge in den Wellenlängenbereich von 170 bis 200 nm und um einen kurzwelligen Anteil der UV-Emission einer UV-Strahlenquelle zu vermindern.

2. Verwendung nach Anspruch 1, **dadurch gekennzeichnet, dass** mindestens der SiO₂-Anteil des dotierten Quarzglases synthetisch erzeugt ist.

3. Verwendung nach Anspruch 1 oder 2, **dadurch gekennzeichnet, dass** das dotierte Quarzglas einen Hydroxylgruppengehalt von weniger als 200 Gew.-ppm, vorzugsweise weniger als 1 Gew.-ppm, aufweist.

4. UV-Lampe mit einem Strahlengang für Arbeitsstrahlung mit Wellenlängen im ultravioletten Spektralbereich, **dadurch gekennzeichnet, dass** im Strahlengang ein Filtermaterial aus dotiertem Quarzglas vorgesehen ist, das zu mindestens 99 Gew.-% aus SiO₂ und Al₂O₃ besteht, wobei der Al₂O₃-Anteil im Bereich von 2 bis 4 Gew.-% liegt, und dessen Kantenwellenlänge im Wellenlängenbereich von 170 bis 200 nm liegt, und dessen spektrale Transmission bei der Wellenlänge von 210 nm 80% mm⁻¹ oder höher ist.

5. UV-Lampe nach Anspruch 4, **dadurch gekennzeichnet, dass** das dotierte Quarzglas einen Hydroxylgruppengehalt von weniger als 200 Gew.-ppm, vorzugsweise weniger als 10 Gew.-ppm, aufweist.

6. UV-Lampe nach Anspruch 4 oder 5, **dadurch gekennzeichnet, dass** ein Strahlaustrittsfenster vorgesehen ist, das aus dem Filtermaterial aus dem Aluminiumoxid-dotierten Quarzglas besteht oder das mit dem Aluminiumoxid-dotierten Quarzglas beschichtet ist.

7. UV-Lampe nach Anspruch 6, **dadurch gekennzeichnet, dass** das Strahlaustrittsfenster auf seiner einem Lampen-Innenraum zugewandten Seite mit einer Schicht aus amorphem Aluminiumoxid belegt ist.

8. UV-Lampe nach einem der Ansprüche 4 bis 7, **dadurch gekennzeichnet, dass** sie als Deuteriumlampe ausgeführt ist.

## Claims

1. The use of doped quartz glass, at least 99% by weight of which consists of SiO₂ and Al₂O₃, wherein the Al₂O₃ fraction is in the range of 2 to 4% by weight, as an optical filter material having a spectral transmittance at the wavelength of 210 nm of 80% mm⁻¹ or higher and an edge wavelength, for the purpose of shifting the edge wavelength into the wavelength range from 170 to 200 nm, and to reduce a short-wave component of the UV emission of a UV radiation source.

2. The use according to claim 1, **characterized in that** at least the SiO₂ fraction of the doped quartz glass is produced synthetically.

3. The use according to claim 1 or 2, **characterized in that** the doped quartz glass has a hydroxyl group content of less than 200 ppm by weight, preferably less than 1 ppm by weight.

4. A UV lamp having a beam path for working radiation with wavelengths in the ultraviolet spectral range, **characterized in that** a filter material of doped quartz glass is provided in the beam path, at least 99% by weight of which doped quartz glass consists of SiO₂ and Al₂O₃, wherein the Al₂O₃ fraction is in the range of 2 to 4% by weight, and its edge wavelength is in the wavelength range of 170 to 200 nm, and its spectral transmittance at the wavelength of 210 nm is 80% mm⁻¹ or greater.

5. The UV lamp according to claim 4, **characterized in that** the doped quartz glass has a hydroxyl group content of less than 200 ppm by weight, preferably less than 10 ppm by weight.

6. The UV lamp according to claim 4 or 5, **characterized in that** a beam exit window is provided which consists of the filter material of the aluminum oxide-doped quartz glass, or which is coated with the aluminum oxide-doped quartz glass.

7. The UV lamp according to claim 6, **characterized in that** the beam exit window is coated with a layer of amorphous aluminum oxide on its side facing toward a lamp interior.

8. The UV lamp according to any one of claims 4 to 7, **characterized in that** it is designed as a deuterium lamp.

## Revendications

1. Utilisation de verre de quartz dopé, lequel est composé d'au moins 99 % en poids de SiO₂ et d'Al₂O₃, la proportion d'Al₂O3 étant située dans la plage de 2 à 4 % en poids, en tant que matériau de filtre optique, lequel présente une transmission spectrale à une longueur d'onde de 210 nm de 80 % mm⁻¹ ou supérieure et une longueur d'onde de bord, dans le but de décaler la longueur d'onde de bord dans la plage de longueurs d'onde de 170 à 200 nm et de réduire la proportion à courte longueur d'onde de l'émission d'UV d'une source de rayonnement UV.

2. Utilisation selon la revendication 1, **caractérisée en ce qu'**au moins la proportion de SiO₂ du verre de quartz dopé est générée synthétiquement.

3. Utilisation selon la revendication 1 ou 2, **caractérisée en ce que** le verre de quartz dopé présente une teneur en groupes hydroxyle inférieure à 200 ppm en poids, de préférence inférieure à 1 ppm en poids.

4. Lampe UV comprenant un trajet de rayonnement pour un rayonnement de travail comprenant des longueurs d'onde dans la plage spectrale ultraviolette, **caractérisée en ce qu'**un matériau de filtre composé de verre de quartz dopé est prévu dans le trajet de rayonnement, lequel est composé d'au moins 99 % en poids de SiO₂ et d'Al₂O₃, la proportion d'Al₂O₃ étant située dans la plage de 2 à 4 % en poids et dont la longueur d'onde de bord est située dans la plage de longueurs d'onde de 170 à 200 nm et dont la transmission spectrale à la longueur d'onde de 210 nm est de 80 % mm⁻¹ ou supérieure.

5. Lampe UV selon la revendication 4, **caractérisée en ce que** le verre de quartz dopé présente une teneur en groupes hydroxyle inférieure à 200 ppm en poids, de préférence inférieure à 10 ppm en poids.

6. Lampe UV selon la revendication 4 ou 5, **caractérisée en ce qu'**une fenêtre de sortie de faisceau est prévue, laquelle est composée du matériau de filtre en verre de quartz dopé avec de l'alumine ou qui est revêtue de verre de quartz dopé avec de l'alumine.

7. Lampe UV selon la revendication 6, **caractérisée en ce que** la fenêtre de sortie de faisceau est recouverte d'une couche en alumine amorphe sur son côté faisant face à un espace intérieur de la lampe.

8. Lampe UV selon l'une quelconque des revendications 4 à 7, **caractérisée en ce qu'**elle est réalisée comme lampe au deutérium.
